(19) Europäisches Patentamt
European Patent Office
Office européen des brevets

(11) **EP 2 428 267 A1**

(12) **EUROPEAN PATENT APPLICATION**

(43) Date of publication:
**14.03.2012 Bulletin 2012/11**

(21) Application number: **10175734.2**

(22) Date of filing: **08.09.2010**

(51) Int Cl.:
*B01J 27/198* (2006.01)   *C07C 253/28* (2006.01)
*C07D 241/24* (2006.01)   *B01J 23/22* (2006.01)
*B01J 23/00* (2006.01)

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **Leibniz-Institut für Katalyse e.V. an der Universität Rostock**
**18058 Rostock (DE)**

(72) Inventors:
• **Dhachapally, Naresh**
  **502334, Andhrapradesh State (IN)**
• **Kalevaru, Venkata Narayana**
  **500013 Andhrapradesh State (IN)**

• **Martin, Andreas**
  **12524 Berlin (DE)**

(74) Representative: **Gulde Hengelhaupt Ziebig & Schneider**
**Patentanwälte - Rechtsanwälte**
**Wallstrasse 58/59**
**10179 Berlin (DE)**

Remarks:
A request for correction of the description has been filed pursuant to Rule 139 EPC. A decision on the request will be taken during the proceedings before the Examining Division (Guidelines for Examination in the EPO, A-V, 3.).

(54) **Catalyst, its preparation and use for the preparation of nitriles from alkyl aromatic or heteroaromatic compounds**

(57)     The present invention relates to a catalyst having the empirical formula $M_aV_bO_x$, wherein M is chosen from a metal selected from the group consisting of Cr, Fe, Co, Ni, Cu, Zn, W, Nb, La, Bi, Mo, Ce, P, Sb, Al, Si and Te; a is 0.001 - 0.999; b is 0.999 - 0.001; and x is determined by the valences of other component elements as well as its use in an ammoxidation reaction of alkyl aromatic hydrocarbons. Subject matter of the present invention is also a method for preparing the ammoxidation catalyst. Especially refers the invention to the use of the catalyst for the preparation of hetero aromatic nitriles by vapour phase ammoxidation. More particularly, the invention relates to the use in a method for preparing 2-cyanopyrazine (CP) from 2-methylpyrazine (MP).

EP 2 428 267 A1

**Description**

The present invention relates to a catalyst and its use in an ammoxidation reaction of alkyl aromatic hydrocarbons. Subject matter of the present invention is also a method for preparing the ammoxidation catalyst. Especially refers the invention to the use of the catalyst for the preparation of hetero aromatic nitriles by vapour phase ammoxidation. More particularly, the invention relates to the use in a method for preparing 2-cyanopyrazine (CP) from 2-methylpyrazine (MP).

Vapour phase ammoxidation (also known as ammonoxidation, oxyamination or oxidative ammonolysis) has become an important technology in industry in the past 50 years. The vapour phase ammoxidation technique is a highly advantageous and a very simple method of producing nitriles in a single step and bears many superior qualities like high purity of the product, higher yields, low cost, continuous process and almost no environmental pollution. The vapour phase ammoxidation technique involves the usage of cheap raw materials such as ammonia and air to convert an active methyl (also aldehyde, alcohol) group into a cyano group in one step using a catalyst. This type of technique can be used to prepare a variety of nitriles such as aliphatic, aromatic and hetero aromatic nitriles from their corresponding substrates in which a methyl moiety, in general, is converted into a cyano moiety.

Hetero aromatic nitriles in particular consist of one or more hetero atoms in the aromatic ring such as nitrogen, oxygen or sulphur. A representative example is 2-cyanopyrazine (CP) with two nitrogen atoms in the ring. In the recent years, there has been an increasing demand for CP due to its high commercial significance. CP is an intermediate to prepare pyrazinamide, which is a generic drug and is used essentially in the treatment of mycobacterium tuberculosis. It is often used in combination with other anti-tuberculosis medications. For instance, pyrazinamide is used in combination with isoniazid and rifampicin in the treatment of tuberculosis (TB). In 2006, WHO launched the new stop TB strategy, where one of the major targets is to completely eliminate TB as a public health problem by 2050. Thus the TB problem requires an urgent attention and there is need to develop potential drugs that are not only cure the disease effectively but also reduces the treatment period. The ammoxidation of 2-methylpyrazine (MP) to CP of the present invention is one such good example for providing an effective TB drug, pyrazinamide.

Methods to prepare 2-cyanopyrazine through vapour phase ammoxidation of 2-methylpyrazine using catalysts are known from the literature.

For instance, US 6 187 943 B1 (2001) discloses the use of a complex multi-component catalytic system comprising $FeSbVCrPCsO_x/SiO_2$ for the ammoxidation of MP. The reaction was carried out using fluidised bed reactors. This type of reactors shows disadvantages including a great demand on mechanical stability of a catalyst against abrasion. In addition, this patent also reports a gradual decrease in the activity with time-on-stream, whereby the conversion of MP and the yield of CP decreased from X-MP = 98.5 % & Y-CP = 78. 9 % (after 2 h of reaction) to X-MP = 91.4 % and Y-CP = 64.3 % (after 10 h of reaction), respectively. In other words, this catalyst seems to suffer from the major problem of deactivation. Other patents e.g. JP 289 962 (2005) using catalysts comprising $Mo-Cr(Sb)/Al_2O_3$ displayed low conversion of MP (65 %) and low yield of CP (59 %). Also US 4 931 561 A (1990) describes relatively low conversion of MP and low yield of CP (X-MP = 87 % & Y-CP = 67 %) by using a $MoPWAlO_x$ catalyst. Such low conversions of MP require additional separation and recycling of unconverted educts. This implies that these two methods still show additional separation or recycling costs. On the whole, it can be stated that all the processes mentioned above possess many disadvantages from industrial production point of view.

*Therefore it is an object of the invention to provide novel catalyst compositions being robust catalysts with good long-term stability and suitable for carrying out the preparation of industrial important nitriles from alkyl aromatic / heteroaromatic hydrocarbons. The invention also aims to supply an easy method for preparing the catalysts. Furthermore, it is an object of the present invention to provide especially an improved vapour phase ammoxidation method, which overcomes many of the above-mentioned disadvantages. In addition, the present invention aims to provide higher conversion rates of the starting compounds for the production of the alkyl aromatic / hetero aromatic nitriles.*

Despite the above noted attempts to provide new and improved catalysts for the preparation of alkyl aromatic hydrocarbons by ammoxidation, the present invention relates to a catalyst comprising a metal vanadate as an active phase. Thus in a first aspect, the present invention provides a catalyst having the empirical formula $M_aV_bO_x$, wherein M is chosen from a metal selected from the group consisting of Cr, Fe, Co, Ni, Cu, Zn, W, Nb, La, Bi, Mo, Ce, P, Sb, Al, Si and Te; a is 0.001 - 0.999; b is 0.999 - 0.001; and x is determined by the valences of other component elements.

The "metal vanadate catalyst" as used herein, refers to a catalyst wherein the catalyst consists of two components, i.e. one selected metal (M), vanadium (V) and oxide (O). The metal vanadate catalyst as used herein refers to a "two-component catalyst".

In contrast to the known multi-catalysts, which comprise multi-components and more complex catalyst compositions consisting of two or more promoters with them, the catalyst of the present invention comprises only two components and represents a very simple system exhibiting better performance. The two components consist of vanadium (V) and second metal oxide, which contains the above-described metals M. That means the said catalyst comprises a metal vanadate (i.e. metal oxide catalyst containing vanadium and a further (second) metal as co-component/promoter) as an active phase. The atomic ratio of vanadium to that of second metal M can vary in the range from 0.001 - 99.999 wt%, and more preferably in the range from 5 to 95 wt%.

The use of the further metal provides some advantages, which include lowering the vanadium concentration, increasing the strength of the catalyst, providing good thermal stability, and also enhancing the lifetime and performance of the catalyst and thus increasing the economy of the use in a process for preparation of alkyl aromatic hydrocarbons. This second metal element M, can act as a promoter. "Promoter elements" are substances, which improve the catalytic activity and selectivity. According to the present invention as defined in the general formula the elements M are selected from Cr, Fe, Co, Ni, Cu, Zn, W, Nb, La, Bi, Mo, Ce, P, Sb, Al, Si and Te. The preferred ratio of metal M : vanadium is 9.9:0.1 to 0.1:9.9, preferably 1:1 to 1:9.

In a more preferred embodiment, the invention provides a catalyst $M_aV_bO_x$, wherein M is selected from the group consisting of Cr, Fe, Nb, La, Al and Bi; a, b and x is determined as above. More preferred M is Nb or La.

The present metal vanadate catalyst can also be supported on different catalyst carriers. Suitable carriers may include alumina, titania, silica, beryllia, zirconia, magnesia, silicon carbide, asbestos, or diatomaceous earth.

Catalysts according to the invention are especially $Cr_{0.5}V_{0.5}O_x$, $Fe_{0.5}V_{0.5}O_x$, $Nb_{0.9}V_{0.1}O_x$, $Nb_{0.75}V_{0.25}O_x$, $Nb_{0.5}V_{0.5}O_x$, $Nb_{0.25}V_{0.75}O_x$, $Nb_{0.1}N_{0.9}O_x$, $La_{0.9}V_{0.1}O_x$, $La_{0.7}V_{0.3}O_x$, $La_{0.5}V_{0.5}O_x$, $La_{0.3}V_{0.7}O_x$, $La_{0.15}V_{0.85}O_x$, $La_{0.1}V_{0.9}O_x$, $La_{0.05}V_{0.95}O_x$, $Al_{0.5}V_{0.5}O_x$, $Bi_{0.5}V_{0.5}O_x$.

In particular, the $Nb_{0.5}V_{0.5}O_x$ or $La_{0.1}V_{0.9}O_x$ catalyst are preferred. In a particularly preferred embodiment, the $La_{0.1}V_{0.9}O_x$ catalyst is preferred.

The inventive two-component catalyst is robust and exhibits good long-term stability, high reactivity and selectivity along with extremely high space-time-yields by ammoxidation of alkyl aromatic hydrocarbons. The new catalyst is especially able for the preparation of hetero aromatic nitriles by ammoxidation of hetero aromatic compounds in a single step, preferably for the preparation of 2-cyanopyrazine (CP) from 2-methylpyrazine (MP). Especially the $La_{0.1}V_{0.9}O_x$ catalyst shows remarkable potentiality and exhibits a conversion of MP, as high as ∼100 % with 86 % molar yield of CP. In addition, the highest space-time-yield of CP (ca. 520 $g_{CP}/kg_{cat}/h$) was also successfully achieved over this $La_{0.1}V_{0.9}O_x$ catalyst.

In another embodiment, the present invention relates to a method for the preparation of the above two-component catalyst comprising the steps of:

- preparation of a vanadium precursor solution from suitable precursors being a vanadium source and appropriate solvents and auxiliary agents,

- preparation of a metal (M) oxide precursor solution (wherein M has the above meaning) from suitable precursors being a metal oxide source and appropriate solvents and auxiliary agents,

- addition of the vanadium precursor solution to the metal precursor solution

- after complete addition heating to dryness (for example removal of excess solvent on a hot plate), drying the formed solid catalyst (for example in an oven) and then calcination under suitable conditions / atmosphere.

This preparation method allows providing the highly active and selective catalyst. This simple method of the catalyst preparation also allows obtaining the catalyst with improved performance and stability.

The preparation of the present catalyst involves the usage of various sources of vanadium compounds. These vanadium sources are selected preferably from the compounds ammonium metavanadate, vanadyl sulphate, vanadyl acetyl acetonate, vanadyl oxalate, vanadyl phosphate, vanadium-containing heteropolyacids, salts of the same or the like. Also, an aqueous hydrogen peroxide solution of vanadium oxide or vanadic acid (vanadate) may be used. The use of ammonium metavanadate ($NH_4VO_3$) as vanadium source is preferred.

The metal oxide sources are selected from compounds, which may include nitrates, oxides, chlorides, oxalates, acetates, sulphates and so on. Preferably used metal oxide sources are compounds selected from $Cr(NO_3)_3.9H_2O$, $Fe(NO_3)_3.9H_2O$, $NH_4NbO(C_2O_4)_2.xH_2O$, $La(NO_3)_3.6H_2O$, $Al(NO_3)_3.9H_2O$ or $Bi(NO_3)_3.6H_2O$.

The synthesis of the precursor solutions is known for the persons skilled in the catalyst preparation art. Starting with the respective vanadium or another metal source, the compound is dissolved in a solvent and by addition of suitable auxiliary agents for the metal and vanadium precursor solutions. The resulting solution can be heated under stirring until a clear solution is obtained. The heating is preferably carried out to a temperature of 50 - 70 °C. The suitable solvents are selected, for example water and some selected organic solvents such as methanol, ethanol, propanol, iso-propanol, butanol, iso-butanol, acetone, ethers and the like.

Preferred auxiliary agents include citric acid, tartaric acid, oxalic acid and the like.

Then the vanadium precursor solution is slowly (for example in drops) added to the metal oxide precursor solution preferably at the above temperature of 50 to 70 °C. After complete addition further heating to dryness is carried out, preferably at temperatures of 70 - 90 °C. The excess solvent can be removed, for example by evaporation on a hot plate. A further drying of the solid by heating and a calcination of the resultant catalyst compound follows. The heating can be carried out for at least 10 hours, preferably between 12 and 30 hours, at temperatures of 100 - 120°C, maybe in an oven. Finally, the resultant solid catalyst is further heated in a calcining atmosphere at a temperature in the range of 300° C to 900° C, preferably in the range of 350° C to 700° C, for a period of 1 to 72 hours. The preferred calcining time is in the range of 2 to 48 hours. The calcination can be done in different atmospheres, which include inert gas ($N_2$, He or Ar), air and reducing gas ($H_2$, CO), preferably air is used at a flow rate of 2-20 l/h, more preferably 3-10 l/h.

As already mentioned the catalyst of the invention is active and selective for the liquid phase or vapour phase ammoxidation of different alkyl aromatic hydrocarbons. The catalyst is especially usable for the ammoxidation of various hetero aromatic hydrocarbons for the preparation of hetero aromatic nitriles.

Therefore, in a further embodiment, the purpose of the present invention is to solve the problems described above in the prior art. The use of the new catalysts provides especially an effective method for the preparation of hetero aromatic nitriles by vapour phase ammoxidation of heteroaromatic compounds at a reaction temperature comprised in the range of 300 to 500 °C in a fixed bed reactor. The hetero aromatic compounds can be mono-, di- or tri-alkylated compounds, preferably mono-alkylated hetero aromatic compounds.

The term "aromatic nitriles" refers to the compounds that are derived from alkyl aromatic hydrocarbons in which a methyl moiety is converted into a cyano moiety. The "hetero aromatic nitriles" are meant aromatic nitriles, which bear one or more hetero atoms in the aromatic ring such as nitrogen, oxygen or sulphur atoms. A particular example is 2-cyanopyrazine.

"Vapour phase ammoxidation" refers to the process wherein an active methyl (also alcohol and aldehyde) moiety is converted into a cyano moiety in one step using a catalyst and using gases like ammonia and air normally in presence of steam.

According to the preferred use of the catalysts the starting compounds are for example "hetero aromatic hydrocarbons". The term "hetero aromatic hydrocarbons" refers to aromatic derivates, which bear one or more hetero atoms, such as nitrogen, oxygen or sulphur atoms. The hetero aromatic compounds contain heterocyclic ring selected preferably from the group consisting of pyridine, piperidine, pyrrole, indole, pyrazine, piperizine, quinoline, isoquinoline, furan, thiophene, thiazole, imadazole and the like. The heterocyclic ring also contain as a side chain one or more alkyl moieties.

The term "alkyl" moiety as used herein means a straight chain or branched-chain saturated hydrocarbon moiety containing 1 to 6 carbon atoms. Examples of suitable alkyl moieties include but are not limited to methyl, ethyl, diethyl, n-propyl, isopropyl, di-isopropyl, acetyl, n-butyl, isobutyl, set-butyl, tert-butyl, pentyl, hexyl, and the like.

The hetero aromatic compound can also contain one or more substituents on the ring such as halogen group, hydroxyl group, alkoxy group, amino group, nitro group, cyano group, phenyl group and the like.

Preferred examples of hetero aromatic compounds include 2-methylpyridine, 3-methylpyridine, 4-methylpyridine, 2,3-dimethylpyridine, 2,4-dimethylpyridine, 2,5-dimethylpyridine, 2,6-dimethylpyridine, 3,4-dimethylpyridine, 3,5-dimethylpyridine, 2-methyl-5-ethylpyridine, 2,4,6-trimethylpyridine, 2-methylpiperidine, 3-methylpiperidine, 4-methylpiperidine, 2-methylpyrazine, 2,5-dimethylpyrazine, 2-methylpiperizine, furfural, 2-methylthiophene, 3-methylthiophene, 4-methylthiazole and the like. These hetero aromatic compounds may be used singly or in combination.

In a more preferred embodiment, the said hetero aromatic compound is 2-methylpyrazine (MP). Thus, the invention relates especially to a method for preparing 2-cyanopyrazine by vapour phase ammoxidation of 2-methylpyrazine in a fixed bed reactor, using the new two-component catalyst.

A "fixed bed reactor" is defined as a reactor provided with a fixed bed wherein a solid catalyst is applied. A catalyst is a substance that increases the rate of a chemical reaction without being consumed in the reaction.

The method according to the invention is carried out at particular reaction conditions. In a preferred embodiment, the method of the present invention is carried out at a reaction temperature comprised in the range of 300 to 500 °C. The optimum temperature depends on several factors such as the concentration of the organic feed, the amount of catalyst used, feed gas composition and the contact time. The reaction pressure may be atmospheric, subatmospheric or super-atmospheric. Preferably the pressure is in the range from atmospheric to 5 bars.

Using the new catalysts the contact time is reduced, which avoid over-oxidation. Furthermore using the catalysts the residence time is very short, 0.01 to 20 seconds, preferably 0.05 to 10 seconds, more preferably 0.1 to 5 seconds. The "residence time" or "contact time" is used herein as synonyms and refer to the time, where the reagents are in contact with the catalyst particles in said reactor. The residence time is defined as the reciprocal of space velocity and the unit is generally seconds.

According to the invention, air, $NH_3$ and $N_2$ are supplied to a fixed bed reactor comprising the new two-component catalyst. The liquid feed (for example MP and water) are injected in said reactor using HPLC pump and vapourised in a preheating zone provided on the top of the catalyst bed.

As already mentioned, in particular, the present invention relates to the preparation of CP by vapour phase ammoxidation of MP at a reaction temperature comprised in the range of 300 to 500 °C in a fixed bed reactor, using a new two-component catalyst. This method for preparing CP results in an improvement of several reaction parameters compared with vapour phase ammoxidation methods known so far, including higher molar yields and extremely higher space-time-yields of CP besides higher conversion rates of MP.

Compared with known methods of CP preparation by vapour phase ammoxidation, the present invention offers improvements in different ways such as

i) providing higher molar yields and

ii) obtaining significantly higher space-time-yields.

iii) In addition, the use of the inventive catalysts provides higher conversion rates of MP. The present invention provides almost total conversion of MP with significantly high molar yield of CP (86 %) along with extremely high space-time-yields of 526 $g_{CP}/Kg_{cat}/h$.

Ammoxidation is considered to be the most promising and potential technique for the production of nitriles from their corresponding hydrocarbons. The preparation of CP is under suitable reaction conditions by vapour phase ammoxidation using cheaply available reactants like air, $NH_3$ and $H_2O$ in addition to MP. Using a new catalyst both the molar yields and space-time-yields for the preparation of CP are improved for economical industrial production.

In the inventive method the molar concentration of MP is preferably in the range from 0.5 to 15 %, more preferably in the range from 1 to 10 %. The mole ratio of $H_2O$ to MP is in the range of 1 to 50, preferably 2 to 20. The mole ratio of $NH_3$ to MP is in the range of 1 to 25, preferably 2 to 15. The mole ratio of air to MP is less than 80 moles, preferably less than 50 moles. The ratio of $O/NH_3$ is preferably in the range between 0.2 to 15 moles, more preferably 0.5 to 10 moles.

Air is generally used as a source of oxygen. However, such air can either be enriched with oxygen or diluted with an adequate diluent gas such as nitrogen, steam or even carbon dioxide. In a preferred embodiment, the air used is synthetic air containing 20.5 % oxygen in it.

The method according to the present invention for preparing CP from MP by vapour phase ammoxidation can be carried out using metal vanadate based catalysts in a fixed bed tubular stainless steel or quartz reactor at temperatures in the range from 300 to 500 °C under normal atmospheric pressure.

The supply of nitrogen gas as a dilution gas aims to maintain constant space velocity during optimization of the process parameters and also to increase gas hourly space velocity (GHSV), or in other words to reduce residence time. The "space velocity" of a gas refers to the volume of a gas measured at a specific temperature and pressure passing through the catalyst bed in a unit time. The "gas hourly space velocity (GHSV)" is defined as the total volume of a gas measured at a specific temperature and pressure passing through the catalyst bed per unit time (usually per hour).

The method of the present invention provides several important advantages compared to methods reported so far in the literature. One advantage includes the possibility of preparing CP in greater yields, i.e. at least 85 mol% yield with almost total conversion of MP (i.e. ca. 100 %). The method provides higher gas hourly space velocities (GHSV) and lower residence times. The GHSV is preferably comprised in the range from 8500 to >15000 h$^{-1}$ and the residence times comprises between 0.2 to 0.5 seconds. Further, the method allows preparing CP at better and higher space-time-yields (STY), which are comprised in the range from 300 to >500 $g_{CP}/kg_{cat}/h$. The "space-time-yield" is defined herein as the quantity of product (CP) obtained per kg of catalyst per unit time (i.e. one hour). To the best of the knowledge of the inventor, the STY showed in the present invention using the new catalysts, is the highest until now compared to all other processes reported so far in the literature regardless of the catalyst system used. In addition, the major end product of the method according to the invention is CP. Only marginal amounts of pyrazine, pyrazinamide and small amounts of total oxidation products, i.e. CO and $CO_2$, are produced (< 5 %). Thus, the method also provides the end product of CP in higher yield.

The invention provides a method wherein the dilution of organic substrate (MP) with water is less than 50 moles, preferably less than 25 moles per each mole of MP fed. $H_2O$ is generally added in the form of a liquid at room temperature but will however be converted into vapour or steam in the preheating zone, whereby the latter is mixed with the feed gas in a fixed proportion. The presence of water vapour plays an important role in controlling the surface metal oxide structures. Moisture is able to interact with oxygen functionalities of the surface vanadia species via hydrogen-bonding. The presence of water vapour is expected to suppress the formation of total oxidation products by blocking the most active sites on vanadium oxide structures. Addition of water vapour also moderates the temperature variation in the reactor. The presence of water vapour is also considered to help the easy desorption of products and also limit the adsorption of reactants due to competitive adsorption. Therefore, mixing of steam with organic feed can significantly enhance the selectivity of desired product.

In another embodiment, the invention provides a method wherein the catalyst is diluted with an inert medium in the ratio of 0.5 to 8.0, preferably in the ratio of 1 to 5 by weight with respect to the weight of said catalyst prior to its loading in the reactor. Since the ammoxidation reaction is exothermic in nature, dilution of the catalyst with an inert medium like porcelain beads, corundum ($Al_2O_3$) particles, quartz beads, glass beads, or the like.

The method according to the present invention can be carried out in a fixed bed stainless steel reactor (9.4 mm i.d. and 250 mm long), and heated in an electrical furnace. Air, $NH_3$ and $N_2$ supplied are commercially available gases from compressed gas cylinders. The premixed liquid feed (i.e. MP and the water) is dosed using HPLC pump and vapourised in a preheating zone provided on the top of the catalyst bed. One gram of catalyst particles mixed with corundum crystals in 1 : 1 dilution by weight may be loaded in the reactor and the reaction is performed. The product stream is collected every one hour and analysed by gas chromatography equipped with FID. The product mixture is collected in an ice-cooled trap containing solvent, which include methanol, ethanol, isopropanol, dichloromethane, or any other suitable solvent. In order to assure that there is no escape of any organic vapour, collection of the product stream in 2 to 3 series of ice cooled traps with any one of the above-mentioned solvents. The total oxidation products (i.e. $CO_x$) are estimated by GC.

The present invention is illustrated in greater detail with reference to the following examples, but it is understood that the present invention is not deemed to be limited thereto. Examples 1, 2 and 3 illustrate the preparation of different metal vanadate catalysts according to the invention. Example 4 describes the experimental set up and catalytic testing procedure. Examples 5, 6, 7 refer to the influence of various metal vanadates on the catalytic performance of catalysts. Example 8 presents the time-on-stream behaviour of the best metal vanadate catalyst. In the following examples, the conversion, yield and selectivity based on MP are illustrated:

$$\text{Conversion (\%) = A/B X 100}$$

where A is the number of moles reacted MP, and

B is the number of moles of MP fed to the reaction zone.

$$\text{Yield (\%) = C/D X 100}$$

where C is the number of moles CP obtained, and

D is the number of moles of MP fed to the reaction zone.

$$\text{Selectivity (\%) = E/F X 100}$$

where E is the number of moles CP obtained, and

F is the number of moles reacted MP.

$$\text{Space-time-yield (g}_{CP}\text{/kg}_{cat}\text{/h) = G/H X h}$$

where G is the amount in grams of CP obtained in 1 hour, and

H is the amount per kilogram of catalyst used.

## Examples

### Example 1

Example 1 illustrates the preparation of bulk metal vanadate catalysts according to the procedure described below.

The preparation of bulk metal vanadates involves two steps. The 1st step deals with the preparation of selected metal solution (see Table 1). The requisite quantities of appropriate metal salts were dissolved in distilled water, to which desired amount of citric acid (mole ratio of metal : citric acid = 3 : 1) was added and then the mixture was stirred at room temperature until clear solution is obtained. The $2^{nd}$ step involves the preparation of vanadium solution using $NH_4VO_3$ (AMV) as a precursor for vanadium. The required amount of AMV was taken in distilled water and suitable amount of oxalic acid (OA) was added to it (mole ratio of AMV : OA = 1 : 1.5). The above solution was then heated to 60 °C on a hot plate and then kept the solution at same temperature 10 minutes under stirring. The vanadium solution turned to dark blue after the addition of oxalic acid.

Afterwards, the above vanadium-oxalic acid solution was added drop wise to the metal-citric acid solution at 60 °C with constant stirring. After complete addition, the mixture was heated to 80 °C and then slowly evaporated to dryness on a hotplate. The solid thus obtained was further dried at 110 °C for 16 h in an oven. Finally, the samples were calcined in air (4 l/h) under the suitable conditions (see Table 1). Using the same procedure, six different metal vanadates were prepared. The composition of such catalysts and the atomic ratios of metal to vanadium and phase composition are described in Table 1.

Table 1

Catalyst composition, calcination conditions, types of precursors used, atomic ratios of co-components and phase composition of various bulk metal vanadates

| S.No. | Catalyst | Calci-nation (°C / h) | Metal precursor | M : V at. ratio (M = Cr, Fe, Nb, La, Al, Bi) | Phase composition* |
|---|---|---|---|---|---|
| 1 | $Cr_{0.5}V_{0.5}O_x$ | 550/4 | $Cr(NO_3)_3.9H_2O$ | 50 : 50 | $CrVO_4$ (major) $Cr_2V_4O_{13}$ (minor) |
| 2 | $Fe_{0.5}V_{0.5}O_x$ | 550/4 | $Fe(NO_3)_3.9H_2O$ | 50 : 50 | $FeVO_4$ (major) |
| 3 | $Nb_{0.5}V_{0.5}O_x$ | 500/12 | $NH_4NbO(C_2O_4)_2.xH_2O$ | 50 : 50 | $NbVO_5$ (major) $V_2O_5$ (minor) |
| 4 | $La_{0.5}V_{0.5}O_x$ | 600 / 15 | $La(NO_3)_3.6H_2O$ | 50 : 50 | $LaVO_4$ (major) |
| 5 | $Al_{0.5}V_{0.5}O_x$ | 600 / 48 | $Al(NO_3)_3.9H_2O$ | 50 : 50 | $AlVO_4$ (minor) $V_2O_5$ (major) |
| 6 | $Bi_{0.5}V_{0.5}O_x$ | 500 / 5 | $Bi(NO_3)_3.6H_2O$ | 50 : 50 | $BiVO_4$ (major) |

* determined by powder X-ray diffraction

It should be noted that the formation of certain amounts of amorphous phases might be possible depending upon the composition of the catalysts. The phases described here are obtained from XRD analysis.

### Example 2

Example 2 depicts the preparation of $Nb_aV_bO_x$, catalysts with varying Nb/V atomic ratios These samples were also prepared in a similar way as that of the procedure described in Example 1. The catalysts having composition, $Nb_aV_bO_x$, (a = 0 to 1 and b = 1 to 0, x is determined by the valences of other component elements) are prepared. Also, ammonium niobium oxalate (ANO) was used as a precursor for Nb, while AMV precursor for vanadium. The prepared catalysts were dried at 110 °C for 16 h and calcined at 500 °C for 12 h in air (4 l/h) except for pure $V_2O_5$ sample. Pure $Nb_2O_5$ (as comparative compound) was obtained by the direct calcination of ANO precursor and calcined under the conditions mentioned above (500°C/12h/air). The source of pure $V_2O_5$ was AMV, which was calcined at 500 °C for 5 h in air. More details of these samples can be seen from Table 2.

Table 2

Catalyst composition, atomic ratios of co-components and phase composition of different niobium vanadates

| S. No. | Catalyst | Nb : V atomic ratio | Phase composition* |
|---|---|---|---|
| 1 | Pure $Nb_2O_5$ | 100 : 0 | $Nb_2O_5$ |
| 2 | $Nb_{0.9}V_{0.1}O_x$ | 90 : 10 | $Nb_2O_5$ (major) |
| 3 | $Nb_{0.75}V_{0.25}O_x$ | 75 : 25 | $Nb_{18}V_4O_{55}$ (major) |
| 4 | $Nb_{0.5}V_{0.5}O_x$ | 50 : 50 | $NbVO_5$ (major), $V_2O_5$ (minor) |
| 5 | $Nb_{0.25}V_{0.75}O_x$ | 25 : 75 | $V_2O_5$ (major) |

(continued)

| Catalyst composition, atomic ratios of co-components and phase composition of different niobium vanadates | | | |
|---|---|---|---|
| S. No. | Catalyst | Nb : V atomic ratio | Phase composition* |
| 6 | $Nb_{0.1}V_{0.9}O_x$ | 10 : 90 | $V_2O_5$ (major) |

* determined by powder X-ray diffraction

It should be noted that the formation of certain amounts of amorphous phases might be possible depending upon the composition of the catalysts. The phases described here are obtained from XRD analysis.

*Example 3*

Example 3 illustrates the preparation of $La_aV_bO_x$ catalysts with varying La/V atomic ratios These samples were prepared again according to the procedure described in Example 1. The catalysts having composition: $La_aV_bO_x$, where a = 0 to 1 and b = 1 to 0, x is determined by the valances of other component elements are prepared. Lanthanum nitrate hexahydrate ($La(NO_3)_3.6H_2O$) (LNH) was used as a precursor for La, while AMV was used as a precursor for vanadium. The prepared catalysts were dried at 110 °C for 16 h and then calcined at 600 °C for 15 h in air (4 I/h). However, pure $La_2O_3$ solid (as comparative compound) was obtained by calcining the LNH precursor under the conditions mentioned above. Again, AMV was the precursor for preparing pure $V_2O_5$, the calcination conditions are similar to the one shown above (i.e. 500°C/5h/air). The details of these samples are given in Table 3.

Table 3

| Catalyst composition, atomic ratios of co-components and phase composition of different lanthanum vanadates | | | |
|---|---|---|---|
| S. No. | Catalyst | La : V atomic ratio | Phase composition* |
| 1 | Pure $La_2O_3$ | 100 : 0 | $La_2O_3$ |
| 2 | $La_{0.9}V_{0.1}O_x$ | 90 : 10 | amorphous |
| 3 | $La_{0.7}N_{0.3}O_x$ | 70 : 30 | $La_{0.33}V_2O_5$ (weak intensity), $LaVO_4$ |
| 4 | $La_{0.5}V_{0.5}O_x$ | 50 : 50 | $LaVO_4$ (major) |
| 5 | $La_{0.3}V_{0.7}O_x$ | 30 : 70 | $LaVO_4$ (major), $V_2O_5$ (minor) |
| 6 | $La_{0.15}V_{0.85}O_x$ | 15 : 85 | $V_2O_5$ (major), $LaVO_4$ (minor) |
| 7 | $La_{0.1}V_{0.9}O_x$ | 10 : 90 | $V_2O_5$ (major), $LaVO_4$ (minor) |
| 8 | $La_{0.05}V_{0.95}O_x$ | 5 : 95 | $V_2O_5$ (major), $LaVO_4$ (minor) |

* determined by powder X-ray diffraction

*Example 4*

Example 4 describes the experimental set up and catalytic testing procedure of ammoxidation reaction carried out according to the invention.

The catalytic testing was conducted in a down flow fixed bed stainless steel reactor (i.d. = 9.4 mm, length = 250 mm) in vapour phase at atmospheric pressure. In a typical experiment, 1 g of the catalyst particles (0.5-0.8 mm size) diluted with equal amount of corundum (catalyst : corundum = 1 : 1 w/w) with same size were loaded in the reactor. The catalyst was suspended between two quartz wool plugs in the middle of the reactor. Also the upper and lower portions of the catalyst bed were filled with corundum. The liquid feed of premixed MP and $H_2O$ mixture (mole ratio of MP : $H_2O$ = 1 : 13) was dosed using HPLC pump and was vaporized in a preheating zone. The flow rates of gases such as $NH_3$, air and $N_2$ are controlled by mass flow controllers. Air, $NH_3$ and $N_2$ (for dilution) supplied were commercially available gases from compressed gas cylinders. The molar ratio of reactant feed mixture is MP : $H_2O$ : $NH_3$ : air : $N_2$ = 1 : 13 : 5-7 : 26-30 : 18-22 (in general). The reaction was carried out at in the temperature range of 340 - 450 °C; GHSV = 8600 to 15800 and $\tau$ = 0.2 to 0.5 s. Two thermocouples were positioned one at the centre of the catalyst bed to indicate reaction temperature and the other one was attached to furnace through temperature indicator cum controller to monitor the temperature of the reactor. The samples were collected every hour under steady sate conditions and analysed by Gas Chromatograph (GC-2014 Shimadzu, Japan) equipped with flame ionization detector (FID). The liquid samples were analysed using FFAP column, while the gases products (i.e. CO and $CO_2$) were analysed by GC using methaniser and GSQ column.

### Example 5

Example 5 describes the conversion of MP, yields of products and space-time-yields of CP during ammoxidation reaction carried out according to the invention. The molar concentration of MP was 1.4 % and the ratio of $O / NH_3$ was 1.5 %. The following catalysts were prepared according to the procedure given in Example 1 and tested according to Example 4 and the results are given in Table 4.

Table 4

Effect of temperature on the catalytic performance of various bulk metal vanadates in the ammoxidation of 2-methylpyrazine

| S. No. | Catalyst | T (°C) | X-MP (%) | Y-CP (%) | Y-PyA (%) | Y-Py (%) | Y-Others (%) | STY ($g_{CP}/kg_{cat}/h$) |
|---|---|---|---|---|---|---|---|---|
| 1 | $Cr_{0.5}V_{0.5}O_x$ | 360 | 89 | 36 | 16 | 16 | 21 | 235 |
| | | 380 | 99 | 42 | 9 | 25 | 13 | 271 |
| | | 400 | 100 | 38 | 5 | 31 | 15 | 248 |
| 2 | $Fe_{0.5}V_{0.5}O_x$ | 360 | 93 | 40 | 18 | 14 | 21 | 289 |
| | | 380 | 99 | 38 | 8 | 22 | 31 | 271 |
| | | 400 | 100 | 36 | 5 | 23 | 36 | 260 |
| 3 | $Nb_{0.5}V_{0.5}O_x$ | 360 | 77 | 46 | 16 | 5 | 10 | 273 |
| | | 380 | 94 | 69 | 15 | 9 | 1 | 440 |
| | | 400 | 100 | 64 | 10 | 14 | 12 | 408 |
| 4 | $La_{0.5}V_{0.5}O_x$ | 380 | 65 | 43 | 5 | 2 | 11 | 282 |
| | | 400 | 81 | 58 | 3 | 5 | 15 | 381 |
| | | 420 | 92 | 62 | 2 | 9 | 19 | 411 |
| | | 440 | 100 | 62 | 1 | 13 | 24 | 411 |
| 5 | $Al_{0.5}V_{0.5}O_x$ | 340 | 95 | 25 | 21 | 38 | 11 | 156 |
| | | 360 | 100 | 18 | 8 | 55 | 19 | 113 |
| | | 380 | 100 | 10 | 4 | 60 | 26 | 63 |
| 6 | $Bi_{0.5}V_{0.5}O_x$ | 360 | 20 | 6 | 3 | 1 | 9 | 38 |
| | | 380 | 30 | 13 | 3 | 2 | 12 | 84 |
| | | 400 | 45 | 20 | 2 | 5 | 18 | 129 |

X-MP = conversion of 2-methylpyrazine; Y-CP = yield of cyanopyrazine; Y-PyA = yield of pyrazinamide; Y-Py = yield of pyrazine; Y-Others = yields of CO, $CO_2$ and unknown products; STY = space-time-yield.

Among various bulk metal vanadates tested, niobium vanadate and lanthanum vanadates exhibited better activity, selectivity and space-time-yields and hence these two systems are further investigated with some modifications in their composition. The results obtained on such investigations are shown below separately.

### Example 6

Example 6 refers to the influence of Nb/V ratio on the catalytic performance of different $Nb_aV_bO_x$ catalysts. The following catalysts were prepared according to the procedure given in Example 2 and tested according to Example 4 at 380 °C temperature (in general) and the results are given below in Table 5.

Table 5

Effect of Nb/V atomic ratio on the catalytic performance of $Nb_aV_bO_x$ catalysts in the ammoxidation of 2-methylpyrazine

| S. No. | Catalyst | X-MP (%) | Y-CP (%) | Y-PyA (%) | Y-Py (%) | Y-others (%) | STY $(g_{CP}/kg_{cat}/h)$ |
|---|---|---|---|---|---|---|---|
| 1 | Pure $Nb_2O_5$ | 18 | 1 | 0 | 5 | 12 | 6 |
| 2 | $Nb_{0.9}V_{0.1}O_x$* | 98 | 22 | 9 | 52 | 15 | 135 |
| 3 | $Nb_{0.75}V_{0.25}O$ | 97 | 51 | 10 | 21 | 15 | 296 |
| 4 | $Nb_{0.5}V_{0.5}O_x$ | 94 | 69 | 15 | 9 | 1 | 440 |
| 5 | $Nb_{0.25}V_{0.75}O_x$ | 100 | 59 | 13 | 21 | 7 | 367 |
| 6 | $Nb_{0.1}V_{0.9}O_x$ | 99 | 56 | 11 | 14 | 18 | 352 |

*T = 360 °C; X-MP = conversion of 2-methylpyrazine; Y-CP = yield of cyanopyrazine; Y-PyA = yield of pyrazinamide; Y-Py = yield of pyrazine; Y-Others = yields of CO, $CO_2$ and unknown products; STY = space-time-yield

From these results, it is obvious that pure $Nb_2O_5$ showed the poor performance. However, combination of Nb and V significantly enhanced the activity and selectivity. Among various $NbVO_x$ solids, the one with Nb/V = 50 : 50 displayed the best performance with considerably good space-time-yields.

*Example 7*

After successful tests on the influence of $Nb_aV_bO_x$ with varying Nb/V ratios, the further studies were focussed on checking the performance of $La_aV_bO_x$ solids. Example 7 describes the influence of La/V ratio on the catalytic performance of different $La_aV_bO_x$ catalysts. The following catalysts were prepared according to the procedure given in Example 3 and tested according to Example 4 at 420 °C temperature (in general) and the results are presented in Table 6.

Table 6

Effect of La/V atomic ratio on the catalytic performance of different $La_aV_bO_x$ catalysts in the ammoxidation of 2-methylpyrazine

| S. No. | Catalyst | X-MP (%) | Y-CP (%) | Y-PyA (%) | Y-Py (%) | Y-others (%) | STY $(g_{CP}/kg_{cat}/h)$ |
|---|---|---|---|---|---|---|---|
| 1 | Pure $La_2O_3$ | 3 | 0 | 0 | 0 | 3 | - |
| 2 | $La_{0.9}V_{0.1}O_x$ | 13 | 0 | 0 | 1 | 12 | - |
| 3 | $La_{0.7}V_{0.3}O_x$ | 33 | 0 | 0 | 7 | 26 | - |
| 4 | $La_{0.5}V_{0.5}O_x$ | 92 | 62 | 2 | 9 | 19 | 411 |
| 5 | $La_{0.3}V_{0.7}O_x$ | 100 | 74 | 4 | 9 | 13 | 451 |
| 6 | $La_{0.15}V_{0.85}O_x$ | 99 | 83 | 6 | 8 | 2 | 506 |
| 7 | $La_{0.1}V_{0.9}O_x$ | 99 | 86 | 3 | 9 | 1 | 517 |
| 8 | $La_{0.05}V_{0.95}O_x$ | 100 | 73 | 4 | 12 | 11 | 453 |

X-MP = conversion of 2-methylpyrazine; Y-CP = yield of cyanopyrazine; Y-PyA = Yield of pyrazinamide; Y-Py = yield of pyrazine; Y-Others = yields of CO, $CO_2$ and unknown products; STY = space-time-yield

As expected, pure $La_2O_3$ is almost inactive. However, the combination of La and V remarkably improved the performance. Among different $La_aV_bO_x$ solids tested, the catalyst with 10 : 90 La/V atomic ratio ($La_{0.1}V_{0.9}O_x$) exhibited the superior performance. The best yield of CP based on MP charged is found to be 86 % at almost total conversion (100%) of MP was achieved. Additionally, the best space-time-yields (STY) of CP ca. 517 $g_{CP}/kg_{cat}/h$ were successfully obtained for the first time. In addition, this is the highest value obtained so far compared to all other existing literature. After obtaining such amazing results on $La_{0.1}V_{0.9}O_x$ catalyst, the long-term stability of this catalyst was also tested and the results are presented below.

*Example 8*

Example 8 illustrates the time-on-stream behaviour of the best $LaVO_x$ solid with La/V atomic ratio of 10 : 90

($La_{0.1}V_{0.9}O_x$). This catalyst was prepared according the procedure given in Example 3 and tested according to the procedure presented in Example 4. The results are shown below in Table 7. It appears that the catalyst exhibited quite consistent performance from 4 hours-on-stream onwards. Afterwards, the best molar yields and STY of CP are maintained through out.

Table 7

Time-on-stream catalytic performance of $La_{0.1}V_{0.9}O_x$ catalyst (T = 420 °C)

| Time (h) | X-MP (%) | Y-CP (%) | Y-PyA (%) | Y-Py (%) | Y-others (%) | STY ($g_{CP}/kg_{cat}/h$) |
|---|---|---|---|---|---|---|
| 1 | 98 | 80 | 3 | 9 | 6 | 480 |
| 2 | 99 | 82 | 4 | 9 | 4 | 495 |
| 3 | 99 | 83 | 4 | 8 | 4 | 506 |
| 4 | 99 | 85 | 3 | 9 | 2 | 511 |
| 5 | 99 | 86 | 4 | 9 | 0 | 517 |
| 6 | 99 | 86 | 3 | 9 | 1 | 517 |
| 7 | 99 | 85 | 3 | 10 | 1 | 511 |
| 8 | 99 | 86 | 3 | 9 | 1 | 517 |
| 9 | 99 | 86 | 3 | 9 | 1 | 517 |
| 10 | 99 | 87 | 3 | 9 | 0 | 526 |

X-MP = conversion of 2-methylpyrazine; Y-CP = yield of cyanopyrazine; Y-PyA = yield of pyrazinamide; Y-Py = yield of pyrazine; Y-Others = yields of CO, $CO_2$ and unknown products; STY = space-time-yield

## Claims

1. Catalyst consisting of a metal vanadate with the empirical formula $M_aV_bO_x$, **characterised in that**

   M is selected from the group consisting of Cr, Fe, Co, Ni, Cu, Zn, W, Nb, La, Bi, Mo, Ce, P, Sb, Al, Si and Te;
   a is 0.001 - 0.999; b is 0.999 - 0.001; and
   x is determined by the valences of the other component elements.

2. Catalyst according to claim 1, **characterised in that** M is selected from the group consisting of Cr, Fe, Nb, La, Al and Bi, preferably Nb or La.

3. Catalyst according to claims 1 or 2, **characterised in that** the ratio of metal M : vanadium is 9.9:0.1 to 0.1:9.9, preferably 1:1 to 1:9.

4. Catalyst according to any of claims 1 to 3 **characterised in that** the catalyst is selected from $Cr_{0.5}V_{0.5}O_x$, $Fe_{0.5}V_{0.5}O_x$, $Nb_{0.9}V_{0.1}O_x$, $Nb_{0.75}V_{0.25}O_x$, $Nb_{0.5}V_{0.5}O_x$, $Nb_{0.25}V_{0.75}O_x$, $Nb_{0.1}V_{0.9}O_x$, $La_{0.9}V_{0.1}O_x$, $La_{0.7}V_{0.3}O_x$, $La_{0.5}V_{0.5}O_x$, $La_{0.3}V_{0.7}O_x$, $La_{0.15}V_{0.85}O_x$, $La_{0.5}V_{0.9}O_x$, $La_{0.05}V_{0.95}O_x$, $Al_{0.5}V_{0.5}O_x$, $Bi_{0.5}V_{0.5}O_x$, preferably $Nb_{0.5}V_{0.5}O_x$ or $La_{0.1}V_{0.9}O_x$.

5. Catalyst according to any of claims 1 to 4, **characterised in that** the catalyst is supported on a carrier preferably a carrier of alumina, titania, silica, beryllia, zirconia, magnesia, silicon carbide, asbestos, or diatomaceous earth.

6. A method for the preparation of a catalyst according to any of claims 1 to 5 comprising the steps of:

   - preparing a vanadium precursor solution using a vanadium source, solvents and auxiliary agents;
   - preparing a metal (M) oxide precursor solution using a metal oxide source, solvents and auxiliary agents, wherein M has the above meaning;
   - addition of the vanadium precursor solution to the metal oxide precursor solution,
   - after complete addition heating to dryness, drying the formed solid catalyst and finally calcination in a calcining atmosphere.

7. Method according to claim 6, **characterised in that** said vanadium source is selected from the group consisting of ammonium metavanadate, vanadyl sulphate, vanadyl acetyl acetonate, vanadyl oxalate, vanadyl phosphate, vanadium-containing heteropolyacid and salts of the same, aqueous hydrogen peroxide solution of vanadium oxide or vanadic acid, preferably ammonium metavanadate ($NH_4VO_3$).

8.  Method according to claim 6, **characterised in that** said metal oxide source is selected from the group consisting of metal nitrates, metal oxides, metal chlorides, metal oxalates, metal acetates and metal sulphates, preferably $Cr(NO_3)_3.9H_2O$, $Fe(NO_3)_3.9H_2O$, $NH_4NbO(C_2O_4)_2.xH_2O$, $La(NO_3)_3.6H_2O$, $Al(NO_3)_3.9H_2O$ or $Bi(NO_3)_3.6H_2O$.

9.  Method according to any of claims 6 to 8, **characterised in that** the calcination step is performed at a temperature in the range of 300 to 900°C for a period of 1 to 72 hours, preferably at a temperature in the range of 350 to 700°C for a period of 2 to 48 hours.

10. Use of a metal vanadate catalyst according to any of claims 1 to 5 for the liquid phase or vapour phase ammoxidation of alkyl aromatic / hetero aromatic hydrocarbons in a reactor.

11. Use of the catalyst according to claim 11 for the preparation of hetero aromatic nitriles by ammoxidation of hetero aromatic compounds at a reaction temperature in the range of 300 to 500 °C wherein the hetero aromatic compounds are mono-, di- or tri-alkylated hetero aromatic compounds, preferably mono-alkylated hetero aromatic compounds.

12. Use of the catalyst according to claim 10 or 11, **characterized in that** the residence time of the catalyst is less than 20 seconds, preferably 0.05 to 10 seconds.

13. Use of the catalyst according to any of claims 10 to 12, for the preparation of 2-cyanopyrazine from 2-methylpyrazine.

14. Use of the catalyst according to claim 13, **characterised in that** the molar concentration of 2-methylpyrazine is 0.5 to 15 % and the molar ratio of $O/NH_3$ in the ammoxidation reaction is 1 to 8.

15. Use of the catalyst according to any of claims 10 to 14, **characterised in that** the catalyst is diluted with an inert medium in the ratio of 0.5 to 8.0 by weight with respect to the weight of said catalyst prior to its addition to the reaction, preferably in an inert medium comprising corundum particles, porcelain beads, quartz beads or glass beads.

**EUROPEAN SEARCH REPORT**

Application Number

EP 10 17 5734

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ITO M ET AL: "A Study on Mixed Vanadium Catalysts for the Ammoxidation of Xylene", BULLETIN OF THE CHEMICAL SOCIETY OF JAPAN, CHEMICAL SOCIETY OF JAPAN, TOKYO, JP, vol. 41, no. 3, 1 January 1968 (1968-01-01), pages 716-721, XP002196027, ISSN: 0009-2673 | 1-4,6-10 | INV. B01J27/198 C07C253/28 C07D241/24 B01J23/22 B01J23/00 |
| A | * the whole document * | 5,11-15 | |
| X | FORNI L: "Ammoxidation of 2-methylpyrazine over oxide catalysts", APPLIED CATALYSIS, vol. 20, no. 1-2, 15 January 1986 (1986-01-15), pages 219-230, XP002631658, DOI: 10.1016/0166-9834(86)80018-5 | 1-5,10, 11,13,14 | |
| Y | * pages 219-224; table 1 * | 15 | |
| A | | 6-9,12 | |
| X | WO 02/16031 A2 (SHOWA DENKO KK [JP]; KURODA YASUSHI [JP]; OHMORI YOKO [JP]; TSUJI KATS) 28 February 2002 (2002-02-28) | 1-10,12 | TECHNICAL FIELDS SEARCHED (IPC) |
| Y | * page 2, line 27 - page 3, line 6 * | 15 | B01J |
| A | * page 9, line 17 - page 11, line 18 * * page 14, line 32 - page 16, line 34 * * examples; compounds catalysts 1-3 * * claims 1-4,8-14 * | 11,13,14 | C07C C07D |
| X | DATABASE WPI Week 200939 Thomson Scientific, London, GB; AN 2009-K06057 XP002631750, -& CN 101 433 836 A (UNIV ZHEJIANG NORMAL [CN]) 20 May 2009 (2009-05-20) | 1-12,15 | |
| A | * abstract * * claims 1,2,4-9; examples 1,3 * | 13,14 | |

-/--

~~The present search report has been drawn up for all claims~~

| Place of search | Date of completion of the search | Examiner |
|---|---|---|
| Munich | 14 April 2011 | Goebel, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 10 17 5734

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE WPI<br>Week 201014<br>Thomson Scientific, London, GB;<br>AN 2010-B33683<br>XP002631751,<br>-& JP 2010 024187 A (MITSUBISHI GAS CHEM CO INC) 4 February 2010 (2010-02-04) | 1-10,12 | |
| A | * abstract *<br>* paragraphs [0006], [0007], [0010], [0013] - [0016], [0018], [0025]; claim 1; examples 1-5 * | 11,13,14 | |
| X | US 3 278 573 A (HUGO KROEPER ET AL)<br>11 October 1966 (1966-10-11) | 1-10,12 | |
| Y | * the whole document * | 15 | |
| A | | 11,13,14 | |
| X | RIZAYEV R G ET AL: "Some fundamental and practical aspects of the ammoxidation of alkylbenzenes",<br>APPLIED CATALYSIS A: GENERAL,<br>vol. 83, no. 2, 21 April 1992 (1992-04-21), pages 103-140, XP002631659,<br>DOI: 10.1016/0926-860X(92)85030-F | 1-12 | |
| | | | TECHNICAL FIELDS SEARCHED (IPC) |
| Y | * page 105 - page 108; table 1 * | 15 | |
| A | * page 130, paragraph 4 - page 131, paragraph 1 * | 13,14 | |
| X | US 3 799 888 A (SEMBAEV D ET AL)<br>26 March 1974 (1974-03-26) | 1-5,10, 11 | |
| Y | * the whole document * | 15 | |
| A | | 6-9, 12-14 | |
| X | GB 1 114 898 A (HALCON INTERNATIONAL INC)<br>22 May 1968 (1968-05-22) | 1-10 | |
| A | * example comparative example * | 11-15 | |

-/--

~~The present search report has been drawn up for all claims~~

| Place of search | Date of completion of the search | Examiner |
|---|---|---|
| Munich | 14 April 2011 | Goebel, Matthias |

EPO FORM 1503 03.82 (P04C01)

Europäisches
Patentamt

European
Patent Office

Office européen
des brevets

# EUROPEAN SEARCH REPORT

Application Number

EP 10 17 5734

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 03/101939 A2 (TESSENDERLO CHEM SA [BE]; MARTIN ANDREAS [DE]; KALEVARU VENKATA NARAYA) 11 December 2003 (2003-12-11) * claims 1-9,12-13 * | 15 | |
| X | DE 20 57 986 A1 (POWER GAS CORP [GB]; ICI LTD [GB]) 16 June 1971 (1971-06-16) | 1-9 | |
| A | * page 23 * * examples 46-47; table VII * * claims 1,5 * | 10-15 | |
| X | POIZOT P ET AL: "Wet-chemical synthesis of various iron(III) vanadates(V) by co-precipitation route", COMPTES RENDUS CHIMIE, vol. 6, no. 1, 13 May 2003 (2003-05-13), pages 125-134, XP002631660, DOI: 10.1016/S1631-0748(03)00015-8 * page 126 * | 1-4 | |
| X | TOUBOUL M ET AL: "Crystal structure of a new form of chromium (III) vanadate (V), CrVO4-I", EUROPEAN JOURNAL OF SOLID STATE AND INORGANIC CHEMISTRY, GAUTHIER-VILLARS, PARIS, FR, vol. 32, 1 January 1995 (1995-01-01), pages 577-588, XP008002372, ISSN: 0992-4361 * abstract * * page 578 * | 1-4,6-9 | TECHNICAL FIELDS SEARCHED (IPC) |

-/--

~~The present search report has been drawn up for all claims~~

| Place of search | Date of completion of the search | Examiner |
|---|---|---|
| Munich | 14 April 2011 | Goebel, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE WPI<br>Week 198103<br>Thomson Scientific, London, GB;<br>AN 1981-03059D<br>XP002631752,<br>& JP 55 145672 A (MITSUWAKA JUNYAKUK)<br>13 November 1980 (1980-11-13)<br>* abstract * | 1-15 | |
| A | NARAYANA K V ET AL: "Cerium fluoride supported V205 catalysts: physico-chemicalcharacterization and 3-picoline ammoxidation activity",<br>JOURNAL OF MOLECULAR CATALYSIS A: CHEMICAL,<br>vol. 223, 18 September 2004 (2004-09-18), pages 321-328, XP4615778,<br>DOI: 10.1016/j.molcata.2004.02.033<br>* the whole document * | 1-15 | |
| A | MARTIN A ET AL: "Heterogeneously Catalyzed Ammoxidation: A Valuable Tool for One-Step Synthesis of Nitriles",<br>CHEMCATCHEM,<br>vol. 2, no. 12,<br>26 August 2010 (2010-08-26), pages 1504-1522, XP002630890,<br>DOI: 10.1002/cctc.201000173<br>* page 1506 - page 1507 *<br>* page 1512 - page 1514; figure 4 *<br>* table 3 * | 1-15 | TECHNICAL FIELDS SEARCHED (IPC) |

~~The present search report has been drawn up for all claims~~

| Place of search | Date of completion of the search | Examiner |
|---|---|---|
| Munich | 14 April 2011 | Goebel, Matthias |

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-15(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 10 17 5734

The Search Division considers that the present European patent application does not comply with the requirements of unity of invention and relates to several inventions or groups of inventions, namely:

1. claims: 1-15(partially)

    insofar as relating to a catalyst comprising a metal vanadate with the empirical formula MaVbOx, *M = Cr*; method for its preparation; its use for the liquid phase or vapour phase ammoxidation of alkyl aromatic / hetero aromatic hydrocarbons

    ---

2. claims: 1-15(partially)

    insofar as relating to a catalyst comprising a metal vanadate with the empirical formula MaVbOx, *M = Fe*; method for its preparation; its use for the liquid phase or vapour phase ammoxidation of alkyl aromatic / hetero aromatic hydrocarbons

    ---

3. claims: 1, 3, 5-15(all partially)

    insofar as relating to a catalyst comprising a metal vanadate with the empirical formula MaVbOx, *M = Co*; method for its preparation; its use for the liquid phase or vapour phase ammoxidation of alkyl aromatic / hetero aromatic hydrocarbons

    ---

4. claims: 1, 3, 5-15(all partially)

    insofar as relating to a catalyst comprising a metal vanadate with the empirical formula MaVbOx, *M = Ni*; method for its preparation; its use for the liquid phase or vapour phase ammoxidation of alkyl aromatic / hetero aromatic hydrocarbons

    ---

5. claims: 1, 3, 5-15(all partially)

    insofar as relating to a catalyst comprising a metal vanadate with the empirical formula MaVbOx, *M = Cu*; method for its preparation; its use for the liquid phase or vapour phase ammoxidation of alkyl aromatic / hetero aromatic hydrocarbons

    ---

6. claims: 1, 3, 5-15(all partially)

    insofar as relating to a catalyst comprising a metal vanadate with the empirical formula MaVbOx, *M = Zn*; method for its preparation; its use for the liquid phase or vapour phase ammoxidation of alkyl aromatic / hetero aromatic

Europäisches
Patentamt
European
Patent Office
Office européen
des brevets

**LACK OF UNITY OF INVENTION
SHEET B**

Application Number

EP 10 17 5734

The Search Division considers that the present European patent application does not comply with the requirements of unity of invention and relates to several inventions or groups of inventions, namely:

       hydrocarbons

                    ---

7. claims: 1, 3, 5-15(all partially)

       insofar as relating to a catalyst comprising a metal
       vanadate with the empirical formula MaVbOx, *M = W*; method
       for its preparation; its use for the liquid phase or vapour
       phase ammoxidation of alkyl aromatic / hetero aromatic
       hydrocarbons

                    ---

8. claims: 1-15(partially)

       insofar as relating to a catalyst comprising a metal
       vanadate with the empirical formula MaVbOx, *M = Nb*; method
       for its preparation; its use for the liquid phase or vapour
       phase ammoxidation of alkyl aromatic / hetero aromatic
       hydrocarbons

                    ---

9. claims: 1-15(partially)

       insofar as relating to a catalyst comprising a metal
       vanadate with the empirical formula MaVbOx, *M = La*; method
       for its preparation; its use for the liquid phase or vapour
       phase ammoxidation of alkyl aromatic / hetero aromatic
       hydrocarbons

                    ---

10. claims: 1-15(partially)

       insofar as relating to a catalyst comprising a metal
       vanadate with the empirical formula MaVbOx, *M = Bi*; method
       for its preparation; its use for the liquid phase or vapour
       phase ammoxidation of alkyl aromatic / hetero aromatic
       hydrocarbons

                    ---

11. claims: 1, 3, 5-15(all partially)

       insofar as relating to a catalyst comprising a metal
       vanadate with the empirical formula MaVbOx, *M = Mo*; method
       for its preparation; its use for the liquid phase or vapour
       phase ammoxidation of alkyl aromatic / hetero aromatic
       hydrocarbons

                    ---

12. claims: 1, 3, 5-15(all partially)

       insofar as relating to a catalyst comprising a metal

| | | LACK OF UNITY OF INVENTION<br>SHEET B | Application Number<br><br>EP 10 17 5734 |
|---|---|---|---|

Europäisches<br>Patentamt<br>European<br>Patent Office<br>Office européen<br>des brevets

The Search Division considers that the present European patent application does not comply with the requirements of unity of invention and relates to several inventions or groups of inventions, namely:

vanadate with the empirical formula MaVbOx, *M = Ce*; method for its preparation; its use for the liquid phase or vapour phase ammoxidation of alkyl aromatic / hetero aromatic hydrocarbons

---

13. claims: 1, 3, 5-15(all partially)

insofar as relating to a catalyst comprising a metal vanadate with the empirical formula MaVbOx, *M = P*; method for its preparation; its use for the liquid phase or vapour phase ammoxidation of alkyl aromatic / hetero aromatic hydrocarbons

---

14. claims: 1, 3, 5-15(all partially)

insofar as relating to a catalyst comprising a metal vanadate with the empirical formula MaVbOx, *M = Sb*; method for its preparation; its use for the liquid phase or vapour phase ammoxidation of alkyl aromatic / hetero aromatic hydrocarbons

---

15. claims: 1-15(partially)

insofar as relating to a catalyst comprising a metal vanadate with the empirical formula MaVbOx, *M = Al*; method for its preparation; its use for the liquid phase or vapour phase ammoxidation of alkyl aromatic / hetero aromatic hydrocarbons

---

16. claims: 1, 3, 5-15(all partially)

insofar as relating to a catalyst comprising a metal vanadate with the empirical formula MaVbOx, *M = Si*; method for its preparation; its use for the liquid phase or vapour phase ammoxidation of alkyl aromatic / hetero aromatic hydrocarbons

---

17. claims: 1, 3, 5-15(all partially)

insofar as relating to a catalyst comprising a metal vanadate with the empirical formula MaVbOx, *M = Te*; method for its preparation; its use for the liquid phase or vapour phase ammoxidation of alkyl aromatic / hetero aromatic hydrocarbons

---

## ANNEX TO THE EUROPEAN SEARCH REPORT
## ON EUROPEAN PATENT APPLICATION NO.

EP 10 17 5734

This annex lists the patent family members relating to the patent documents cited in the above-mentioned European search report.
The members are as contained in the European Patent Office EDP file on
The European Patent Office is in no way liable for these particulars which are merely given for the purpose of information.

14-04-2011

| Patent document cited in search report | | Publication date | Patent family member(s) | | Publication date |
|---|---|---|---|---|---|
| WO 0216031 | A2 | 28-02-2002 | AU | 7879201 A | 04-03-2002 |
| CN 101433836 | A | 20-05-2009 | NONE | | |
| JP 2010024187 | A | 04-02-2010 | NONE | | |
| US 3278573 | A | 11-10-1966 | BE | 632702 A | |
| | | | DE | 1172253 B | 18-06-1964 |
| | | | FR | 1357429 A | 03-04-1964 |
| | | | GB | 972122 A | 07-10-1964 |
| | | | NL | 134411 C | |
| | | | NL | 293050 A | |
| US 3799888 | A | 26-03-1974 | GB | 1317064 A | 16-05-1973 |
| GB 1114898 | A | 22-05-1968 | BE | 668043 A | 09-02-1966 |
| | | | CH | 448989 A | 31-12-1967 |
| | | | DE | 1286002 B | 02-01-1969 |
| | | | IL | 24162 A | 30-07-1969 |
| | | | LU | 49318 A1 | 13-02-1967 |
| WO 03101939 | A2 | 11-12-2003 | AU | 2003242614 A1 | 19-12-2003 |
| | | | EP | 1515946 A2 | 23-03-2005 |
| | | | JP | 2005532344 A | 27-10-2005 |
| | | | US | 2005176984 A1 | 11-08-2005 |
| DE 2057986 | A1 | 16-06-1971 | NONE | | |
| JP 55145672 | A | 13-11-1980 | JP | 1434886 C | 07-04-1988 |
| | | | JP | 62041509 B | 03-09-1987 |

**EP 2 428 267 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6187943 B1
- JP 289962 A
- US 4931561 A